(19)

Europäisches
Patentamt
European
Patent Office
Office européen
des brevets

(11) **EP 1 664 178 B1**

(12) **EUROPEAN PATENT SPECIFICATION**

(45) Date of publication and mention
of the grant of the patent:
**29.10.2008 Bulletin 2008/44**

(51) Int Cl.:
***C08K 5/103*** *(2006.01)* ***C07C 69/76*** *(2006.01)*
***C08L 27/06*** *(2006.01)*

(21) Application number: **04774566.6**

(22) Date of filing: **09.09.2004**

(86) International application number:
**PCT/KR2004/002307**

(87) International publication number:
**WO 2005/023926 (17.03.2005 Gazette 2005/11)**

(54) **DIETHYLENEGLYCOL ESTER BASED PLASTICIZER COMPOSITION AND POLYVINYL CHLORIDE RESIN USING THE SAME**

WEICHMACHERZUSAMMENSETZUNG AUF DIETHYLENGLYKOLESTERBASIS UND POLYVINYLCHLORIDHARZ DAMIT

COMPOSITION DE PLASTIFIANT A BASE D'ESTER DE DIETHYLENEGLYCOL ET RESINE DE POLYCHLORURE DE VINYLE L'UTILISANT

(84) Designated Contracting States:
**AT BE BG CH CY CZ DE DK EE ES FI FR GB GR HU IE IT LI LU MC NL PL PT RO SE SI SK TR**

(30) Priority: **09.09.2003 KR 2003063160**

(43) Date of publication of application:
**07.06.2006 Bulletin 2006/23**

(73) Proprietor: **LG Chem, Ltd.
Seoul 150-721 (KR)**

(72) Inventors:
• **LEE, Kye-Seok,
8-46 LG Chemical Sataek
Daejeon-city 305-340 (KR)**
• **LEE, Kyu-Il,
c/o LG Chem Ltd.,
Daejeon, 305-738 (KR)**
• **KIM, Hyun-Kyu
Daejeon-city 305-728 (KR)**
• **HONG, Chil-Eui
Daejeon-city 305-340 (KR)**
• **GONG, Dong-Hwa,
209-1005 Jugong apt.
Dong-gu,
Daejeon-city 300-131 (KR)**

• **KIM, Hyun-Ju
Daejeon-city 305-340 (KR)**

(74) Representative: **Goddar, Heinz J.
Forrester & Boehmert
Pettenkoferstrasse 20-22
80336 München (DE)**

(56) References cited:
**WO-A1-02/083621** **US-A- 2 585 448**

• **DATABASE CAPLUS [Online] 11 October 2004 BAILEY A.V. ET AL.: 'Preparation of some mixed diesters of aliphatic diols and theri evaluation as plasticizers', XP002998354 Retrieved from STN Database accession no. 1976:463892 & JOURNAL OF THE AMERICAL OIL CHEMISTS SOCIETY vol. 53, no. 5, 1976, pages 176 - 178**
• **DATABASE CAPLUS [Online] 11 October 2004 MAMEDOV SH. A. ET AL.: 'Synthesis and study of chemical transformations of monocaprylhydroxydiethylene glycol', XP002998355 Retrieved from STN Database accession no. 1972:24645 & AZERBAIDZHANSKII KHIMICHESKII ZHURNAL vol. 1, 1971, pages 67 - 72**

EP 1 664 178 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a diethyleneglycol ester based plasticizer composition, and more particularly to a diethyleneglycol ester based plasticizer composition which can be used as plasticizer of a polyvinyl chloride (PVC) resin.

The plasticizer added to a polyvinyl chloride resin or other polymer resins is an essential additive offering the polymer resin physical properties and capabilities such as processability, flexibility, electrical insulation, adhesivity, etc. Conventionally, phthalates, adipates, etc. have been typically used as plasticizer. The most widely used plasticizers are di-2-ethylhexylphthalate (DEHP) and di-2-ethylhexyladipate (DEHA). These plasticizers are used as standards for evaluating other plasticizers.

However, the Environmental Protection Agency (EPA) of the U.S. and the National Institute of Health Sciences of Japan have classified phthalates and adipates as environmental hormone materials.

Thus, development of a plasticizer comprising neither phthalate nor adipate is required. U.S. Patent No. 5,746,783 discloses a diesel fuel additive comprising a diethyleneglycol ester based compound, which comprises netither phthalate structure nor adipate structure and reduces nitrogen oxide exhausted from diesel engine.

A polyvinyl chloride resin is prepared from vinyl chloride monomers and other monomers copolymerizable with the vinyl chloride monomers. By adding such additives as a plasticizer, a stabilizer, a filler, a pigment, etc., the resin can be imbued with a variety of processing properties.

The polyvinyl chloride resin is used for numerous applications including pipes, cables, artificial leather, wallpaper, gloves, toys, wrap films, etc.

Particularly, the polyvinyl chloride resin used for a wrap film for packing foods requires good tensile strength, hardness, elongation capable of wrapping well packing manufactures, transparency capable of seeing well with the naked eye foods, adhesivity after foods packing, transfer resistance, and compatibility with other resins.

It is an aspect of the present invention to provide a diethylene glycol ester compound for a plasticizer not releasing environmental hormone.

It is another aspect of the present invention to provide a diethylene glycol ester based plasticizer composition useful for a plasticizer of a polyvinyl chloride resin comprising a diethylene glycol ester compound.

It is still another aspect of the present invention to provide a polyvinyl chloride resin for a wrap film having superior tensile strength, elongation, hardness, transfer resistance, transparency, and adhesivity using the diethylene glycol ester based plasticizer composition.

In order to attain the aspects, a diethylene glycol ester compound for a plasticizer represented by Formula 1-1 below is provided:

$$R_1OCO\text{-}(CH_2)_2\text{-}O\text{-}(CH_2)_2\text{-}OCOR_2 \qquad (1\text{-}1)$$

wherein $R_1$ is a phenyl group and $R_2$ is an alkyl group having 3 to 12 carbon atoms.

Also a provided is a diethylene glycol ester based plasticizer composition comprising the diethylene glycol ester compound represented by Formula 1-1.

The composition further comprises a diethylene glycol ester compound represented by Formula 1-2 below; and a diethylene glycol ester compound represented by Formula 1-3 below.

$$R_3OCO\text{-}(CH_2)_2\text{-}O\text{-}(CH_2)_2\text{-}OCOR_4 \qquad (1\text{-}2)$$

(wherein each of $R_3$ and $R_4$ is an alkyl group having 3 to 12 carbon atoms.)

$$R_5OCO\text{-}(CH_2)_2\text{-}O\text{-}(CH_2)_2\text{-}OCOR_6 \qquad (1\text{-}3)$$

(wherein each of $R_5$ and $R_6$ is a phenyl group.)

The plasticizer composition of the present invention comprises:

(a) 10 to 80 wt% of the diethylene glycol ester compound represented by Formula 1-1;

(b) 5 to 80 wt% of the diethylene glycol ester compound represented by Formula 1-2; and

(c) 5 to 60 wt% of the diethylene glycol ester compound represented by Formula 1-3 as characterized in the appended claims.

The present invention also provides a method of preparing a diethylene glycol ester based plasticizer composition by esterifiying 10 to 40 wt% of diethylene glycol, 20 to 70 wt% of 2-ethylhexanoic acid, 10 to 60 wt% of benzoic acid, 1 to 10 wt% of xylene, and 0.01 to 1 wt% of tetra-isopropyl titanate at 220 °C for 4 to 10 hours.

The present invention also provides a polyvinyl chloride resin prepared using the diethylene glycol ester based plasticizer composition.

## Advantageous Effects

As described above, the diethylene glycol ester based plasticizer composition of the present invention improves tensile strength, elongation, transfer resistance, hardness, transparency, adhesivity, and/or compatibility of a polyvinyl chloride resin without generating environmental hormones, when employed as the plasticizer of the resin.

## Brief Description of the Drawings

FIG. 1 shows a gas chromatography mass spectrometer (GC-MS) for diethylene glycol ester based plasticizer composition of Example 1 according to the present invention.

FIG. 2 shows a gas chromatography mass spectrometer (GC-MS) for diethylene glycol ester based plasticizer composition of Example 2 according to the present invention.

FIG. 3 shows a gas chromatography mass spectrometer (GC-MS) for diethylene glycol ester based plasticizer composition of Example 3 according to the present invention.

## Best Mode for Carrying Out the Invention

Hereinafter, the present invention is described in more detail.

The present invention is characterized by a plasticizer composition not comprising phthalate or adipate, which are known as environmental hormone materials and having superior tensile strength, elongation, hardness, transfer resistance, transparency and adhesivity, thereby being useful for a polyvinyl chloride resin for a wrap film for packing foods.

The diethylene glycol ester based plasticizer composition of the present invention comprises three selected from the group consisting of diethylene glycol ester compounds represented by Formula 1 below, as characterized in the appended claims:

$$R_7OCO\text{-}(CH_2)_2\text{-}O\text{-}(CH_2)_2\text{-}OCOR_8 \qquad (1)$$

wherein each of $R_7$ and $R_8$ is a phenyl group or an alkyl group having 3 to 12 carbon atoms.

A wrap film used for packing foods should have superior tensile strength, hardness, transfer resistance, transparency, elongation, and adhesivity. A plasticizer is added to the resin formed the foundation of the wrap film to offer such physical properties. The present invention uses the compound represented by Formula 1 as plasticizer.

The plasticizer composition of the present invention offers superior tensile strength, elongation, hardness, transfer resistance, transparency, and adhesivity and has superior processability including compatibility with polyvinyl chloride resin by using two or more diethylene glycol ester compounds selected from the compounds represented by Formula 1.

Of such compounds represented by Formula 1, the present invention is characterized by a diethylene glycol ester compound having the structure of Formula 1-1 below:

$$R_1OCO\text{-}(CH_2)_2\text{-}O\text{-}(CH_2)_2\text{-}OCOR_2 \qquad (1\text{-}1)$$

wherein $R_1$ is a phenyl group and $R_2$ is an alkyl group having 3 to 12 carbon atoms, the compound being benzoic acid 2-{2-(2-ethylhexanoyloxy)-ethoxy}-ethyl ester.

Because the compound represented by Formula 1-1 is the most preferable in terms of stability, a plasticizer composition comprising it has superior physical properties as plasticizer of a polyvinyl chloride resin.

Accordingly, the present invention is characterized by a plasticizer composition of a polyvinyl chloride resin comprising the diethylene glycol ester compound represented by Formla 1-1.

The composition of the present invention also comprises the diethylene glycol ester compounds represented by Formulas 1-2 and 1-3 below as the compound represented by Formula 1:

$$R_3OCO\text{-}(CH_2)_2\text{-}O\text{-}(CH_2)_2\text{-}OCOR_4 \qquad (1\text{-}2)$$

wherein each of $R_3$ and $R_4$ is an alkyl group having 3 to 12 carbon atoms.

$$R_5OCO-(CH_2)_2-O-(CH_2)_2-OCOR_6 \qquad (1\text{-}3)$$

wherein each of $R_5$ and $R_6$ is a phenyl group, wherein,

the plasticizer composition of the present invention comprises:

(a) 10 to 80 wt% of the diethylene glycol ester compound represented by Formula 1-1;

(b) 5 to 80 wt% of the diethylene glycol ester compound represented by Formula 1-2; and

(c) 5 to 60 wt% of the diethylene glycol ester compound represented by Formula 1-3, and wherein

(a) the compound represented by Formula 1-1 is benzoic acid 2-{2-(2-ethylhexanoyloxy)-ethoxy}-ethyl ester,

(b) the compound represented by Formula 1-2 is 2-ethylhexanoic acid 2-{2-(2-ethylhexanoyloxy)-ethoxy}-ethyl ester,

And (c) the compound represented by Formula 1-3 is benzoic acid 2-{2-(benzoyloxy)-ethoxy}-ethyl ester.

Preferably, the diethylene glycol ester based plasticizer composition of the present invention comprises 30 to 60 wt% of benzoic acid 2-{2-(2-ethylhexanoyloxy)-ethoxy}-ethyl ester; 10 to 60 wt% of 2-ethylhexanoic acid 2-{2-(2-ethylhexanoyloxy)-ethoxy}-ethyl ester; and 5 to 50 wt% of benzoic acid 2-{2-(benzoyloxy)-ethoxy}-ethyl ester.

Most preferably, the composition of the present invention comprises the compounds (a), (b), and (c) at a proportion of 40:40:20 based on weight.

Hereinafter, the preparing method of the diethylene glycol ester based plasticizer composition of the present invention is described in more detail.

(a) The benzoic acid 2-{2-(2-ethylhexanoyloxy)-ethoxy}-ethyl ester may be synthesized from diethylene glycol, 2-ethylhexanoic acid and benzoic acid by esterification.

(b) The 2-ethylhexanoic acid 2-{2-(2-ethylhexanoyloxy)-ethoxy}-ethyl ester may be synthesized from diethylene glycol and 2-ethylhexanoic acid by esterification.

And, (c) the benzoic acid 2-{2-(benzoyloxy)-ethoxy}-ethyl ester may be synthesized from diethylene glycol and benzoic acid by esterification.

To sum up, the diethylene glycol ester based plasticizer composition of the present invention may be prepared from diethylene glycol, benzoic acid, and 2-ethylhexanoic acid. In addition, other additives may be used.

In a preferred embodiment of the present invention, the diethylene glycol ester plasticizer composition is prepared as follows.

10 to 40 wt% of diethylene glycol, 20 to 70 wt% of 2-ethylhexanoic acid, 10 to 60 wt% of benzoic acid, 1 to 10 wt% of xylene as entrainer, and 0.05 to 1 wt% of tetra-isopropyl titanate as catalyst, are put in a flask equipped with a stirrer and a condenser. The temperature of the flask is raised to 220 °C and esterification is performed for 4 to 10 hours. After the esterification is over, the unreacted acid is removed with a vacuum pump and the remaining acid is neutralized with 5 to 15 wt% of sodium hydroxide. After washing with water, dehydrating, and filtering, a diethylene glycol ester plasticizer composition comprising 30 to 60 wt% of benzoic acid 2-{2-(2-ethylhexanoyloxy)-ethoxy} ethyl ester, 10 to 60 wt% of 2-ethylhexanoic acid 2-{2-(2-ethylhexanoyloxy)-ethoxy}-ethyl ester, and 5 to 40 wt% of benzoic acid 2-{2-(benzoyloxy)-ethoxy} ethyl ester is obtained.

The resultant diethylene glycol ester plasticizer composition can be used as plasticizer of a polyvinyl chloride resin to improve tensile strength, elongation, hardness, transfer resistance, transparency, adhesivity, and/or compatibility of the resin.

The diethylene glycol ester based plasticizer composition of the present invention may also be used for other resins to improve tensile strength, elongation, transfer resistance, hardness, transparency, and/or adhesivity, and it is particularly suitable for a polyvinyl chloride resin of a wrap film used for packing foods. It may also be used for a polyethylene based foaming sheet.

Thus, the present invention provides a polyvinyl chloride resin using the above-described diethylene glycol ester based plasticizer composition.

The method of preparing the polyvinyl chloride resin is not particularly limited. It can be prepared by the conventional method of copolymerizing polyvinyl chloride with monomers copolymerizable with polyvinyl chloride using the plasticizer composition.

Hereinafter, the present invention is described in more detail through examples. However, the following examples are only for the understanding of the present invention and they do not limit the present invention.

**EXAMPLES**

[Example 1]

(1) Preparation of diethylene glycol ester plasticizer composition

3 moles of diethylene glycol, 5.4 moles of 2-ethylhexanoic acid, 2.4 moles of benzoic acid, 60 g of xylene as entrainer, and 2 g of tetraisopropyl titanate as catalyst, were put in a 2-L round flask equipped with a stirrer and a condenser. The temperature was raised to 220 °C and esterification was performed for 10 hours.

Unreacted acid was removed with a vacuum pump at 220 °C and 2 mmHg, and remaining acid was neutralized with 10 wt% of sodium hydroxide. After washing with water and dehydrating, an absorbent was added. The resultant solution was filtered to obtain a diethylene glycol ester composition.

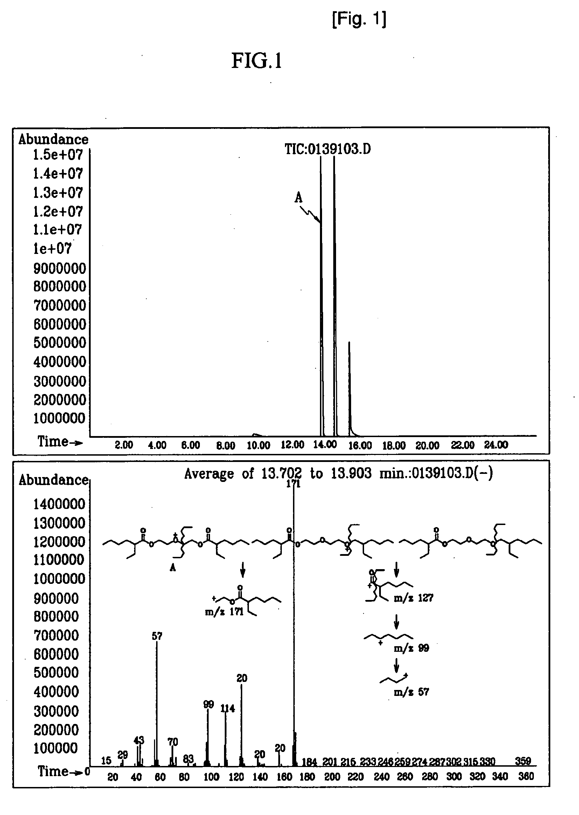

The diethylene glycol ester composition comprised 45 wt% of benzoic acid 2-{2-(2-ethylhexanoyloxy)-ethoxy} ethyl ester, 42 wt% of 2-ethylhexanoic acid 2-{2-(2-ethylhexanoyloxy)-ethoxy}-ethylester, 12 wt% of benzoic acid 2-{2-(benzoyloxy)-ethoxy} ethyl ester, and 1 wt% of other unreacted materials. The composition and the content were confirmed with a gas chromatography mass spectrometer (GC-MS). The GC-MS result is shown in FIG. 1.

(2) Preparation of polyvinyl chloride resin using diethylene glycol ester plasticizer composition

36 parts by weight of the diethylene glycol ester composition prepared in (1), 1.3 part by weight of a calcium-zinc stabilizer (KCZ-08, Kolon) and 12 parts by weight of epoxylated soybean oil (ShinDongBang) per 100 parts by weight of a polyvinyl chloride resin (LS100S, LG Chem) were mixed and prepared sheet a thickness of 5-mm using a roll mill at 165 °C for 3 ninutes. The sheet was pressed to a thickness of 1-mm. The pressing conditions included preheating at 185 °C for 3 minutes, heating for 3 minutes, and cooling for 3 minutes. The resultant sheet having a thickness of 1-mm was prepared into several type C dumbbell shaped samples.

(3) Physical property test of polyvinyl chloride resin

Tensile strength, elongation, hardness, transfer resistance, transparency, and adhesivity were evaluated for the samples prepared in (2). The result is given in Table 1 below.

[0078] * Tensile strength: Tested according to ASTM D638 using a U.T.M. The sample was pulled at a cross head speed of 500 mm/min. The site where the sample was cut was checked and the tensile strength was calculated by the following equation.

[Equation 1]

$$\text{Tensile strength (kgf/mm}^2\text{)} = \text{load (kgf) / (thickness (mm)} \times \text{width (mm))}$$

[0080] * Elongation: Tested according to ASTM D638 using a U.T.M. The sample was pulled at a cross head speed of 500 mm/min. The site where the sample was cut was checked and the elongation was calculated by the following equation.

[Equation 2]

$$\text{Elongation (\%)} = \text{(extension / initial strength)} \times 100$$

[0082] * Hardness: Hardness was tested at 5 sites for each sample to obtain the mean value.

[0083] * Transfer resistance: The initial weight ($W_i$) of the sample was weighed to the fourth decimal point. The sheet sample (3 cm 及 3 cm) was inserted between polystyrene (PS) plates and put in an oven of 80 °C. After letting for 48 hours under a load of 1 kg, the sample was taken out of the oven and kept in a constant temperature bath for at least 4 hours. The weight ($W_o$) of the sample was weighed and the degree of transfer was calculated by the following equation.

[Equation 3]

$$\text{Degree of transfer (\%)} = (W_i - W_o) / W_i \times 100$$

[0085] * Transparency: If the transparency was better than that of the di-2-ethylhexyladipate (DEHA), a standard plasticizer, when observed with naked eyes, the transparency was evaluated as superior. If the transparency was comparable to that of DEHA, it was evaluated as comparable, and if it was inferior to that of DEHA, it was evaluated as inferior.

[0086] * Adhesivity: If the adhesivity was better than that of DEHA, when touched with a hand, the adhesivity was evaluated as superior. If the adhesivity was comparable to that of DEHA, it was evaluated as comparable, and if it was inferior to that of DEHA, it was evaluated as inferior.

[Example 2]

A diethylene glycol ester composition was prepared in the same manner of Example 1, except for changing the composition and content as shown in Table 1. A polyvinyl chloride resin was prepared using the composition as plasticizer. Tensile strength, elongation, hardness, transfer resistance, transparency, and adhesivity were evaluated as in Example 1. The result is given in Table 1.

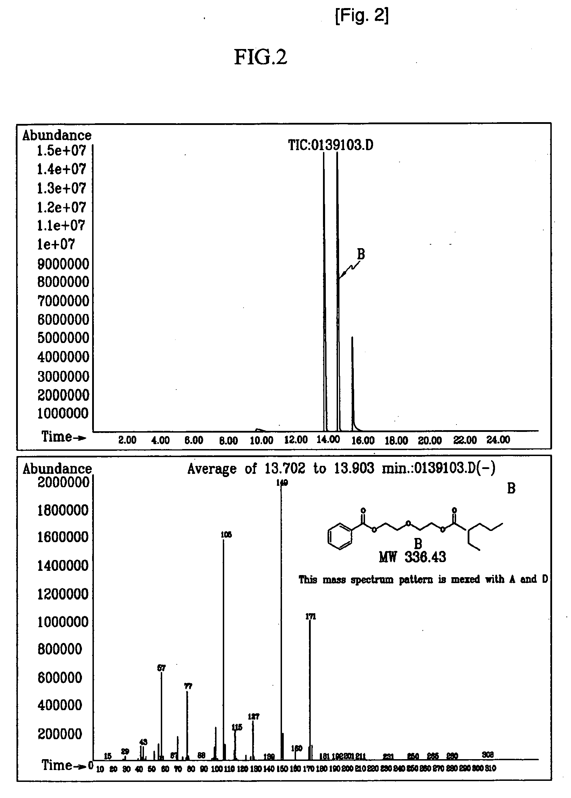

The diethylene glycol ester composition comprised 50 wt% of benzoic acid 2-{2-(2-ethylhexanoyloxy)-ethoxy}-ethyl ester, 24 wt% of 2-ethylhexanoic acid 2-{2-(2-ethylhexanoyloxy)-ethoxy}-ethyl ester, 24 wt% of benzoic acid 2-{2-(benzoyloxy)-ethoxy}-ethyl ester, and 2 wt% of other unreacted materials. The composition and the content were confirmed with a gas chromatography mass spectrometer (GC-MS). The GC-MS result is shown in FIG. 2.

[Example 3]

A diethylene glycol ester composition was prepared in the same manner of Example 1, except for changing the composition and content as shown in Table 1. A polyvinyl chloride resin was prepared using the composition as plasticizer. Tensile strength, elongation, hardness, transfer resistance, transparency, and adhesivity were evaluated as in Example 1. The result is given in Table 1.

The diethylene glycol ester composition comprised 42 wt% of benzoic acid 2-{2-(2-ethylhexanoyloxy)-ethoxy}-ethyl ester, 12 wt% of 2-ethylhexanoic acid 2-{2-(2-ethylhexanoyloxy)-ethoxy}-ethyl ester, 44 wt% of benzoic acid 2-{2-(benzoyloxy)-ethoxy} ethyl ester, and 2 wt% of other unreacted materials. The composition and the content were confirmed with a gas chromatography mass spectrometer (GC-MS).

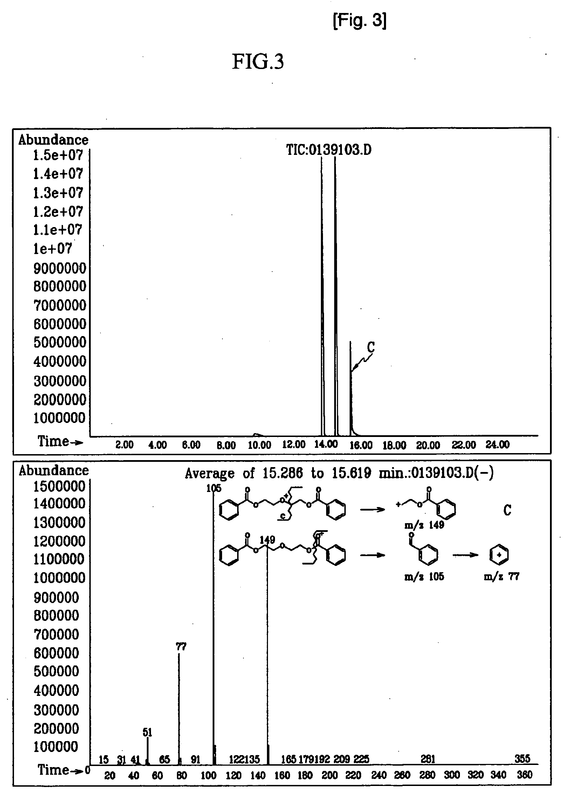

The GC-MS result is shown in FIG. 3.

[Comparative Example 1]

A polyvinyl chloride resin was prepared in the same manner of Example 1, except for using di-2-ethylhexyladipate (DOA, LG Chem) instead of diethylene glycol ester of the present invention as plasticizer. Tensile strength, elongation, hardness, transfer resistance, transparency, and adhesivity were evaluated. The result is given in Table 1.

[Comparative Example 2]

A polyvinyl chloride resin was prepared in the same manner of Example 1, except for using an ester based plasticizer prepared from neopentylglycol and 2-ethylhexanoic acid instead of diethylene glycol ester of the present invention as plasticizer. Tensile strength, elongation, hardness, transfer resistance, transparency, and adhesivity were evaluated. The result is given in Table 1.

[Comparative Example 3]

A polyvinyl chloride resin was prepared in the same manner of Example 1, except for using an ester based plasticizer prepared from triethylene glycol and 2-ethylhexanoic acid instead of diethylene glycol ester of the present invention as plasticizer. Tensile strength, elongation, hardness, transfer resistance, transparency, and adhesivity were evaluated. The result is given in Table 1.

[Comparative Example 4]

A polyvinyl chloride resin was prepared in the same manner of Example 1, except for using diisonoyladipate (DINA, LG Chem) instead of diethylene glycol ester of the present invention as plasticizer. Tensile strength, elongation, hardness, transfer resistance, transparency, and adhesivity were evaluated. The result is given in Table 1.

Table 1

| | | Example | | | Comparative Example | | | |
|---|---|---|---|---|---|---|---|---|
| | | 1 | 2 | 3 | 1 | 2 | 3 | 4 |
| Composition | Com Diethylene positi glycolmol (g) | 3(666) | 3(666) | 3(666) | - | - | - | - |
| | 2-Ethylhex anoic acidmol (g) | 5.4(1.29 6) | 3.3(792) | 2.1(504 | - | 3.1(744 ) | 2.2(528) | - |
| | Benzoic acidmol (g) | 2.4(446. 4) | 3.3(613. 8) | 4.5(837) | - | - | - | - |
| | Neopentylglycolmol (g) | - | - | - | - | 1.2(1241.8) | - | - |
| | Triethylene glycolmol (g) | - | - | - | - | - | 1(150) | - |
| | Xylene (g) | 60 | 60 | 60 | - | 60 | 60 | - |
| | Tetraisopropyl titanate (g) | 2 | 2 | 2 | - | 1.5 | 2 | - |
| Tensile strength (kgf/mm$^2$) | | 191 | 1.81 | 1.85 | 1.81 | 1.87 | 1.84 | 1.73 |
| Elongation (%) | | 390 | 382 | 380 | 380 | 385 | 348 | 371 |
| Transfer resistance (%) | | 195 | 2.21 | 3.22 | 0.2 | 0.2 | 0.3 | 0.1 |
| Hardness (R-scale) | | 75 | 75 | 75 | 78 | 76 | 78 | 76 |
| Transparency | | Superior | Superior | Superior | Standard | Comparable | Inferior | Comparable |
| Adhesivity | | Superior | Comparable | Comparable | Standard | Inferior | Inferior | Inferior |

As seen in Table 1, the polyester resin using the diethylene glycol ester composition of the present invention as plasticizer (Example 1) showed better tensile strength, elongation, transparency, and adhesivity and comparable hardness, compared with those prepared using the DOA (Comparative Example 1), the conventional plasticizer most widely used in a wrap film for packing floods, the DINA (Comparative Example 4), which has the structure similar to that of DEHA, the standard plasticizer, the single-component plasticizer prepared from neopentylglycol and 2-ethylhexanoic acid (Comparative Example 2) and the single-component plasticizer prepared from triethylene glycol and 2-ethylhexanoic acid (Comparative Example 3). Thus, the plasticizer of Example 1 is suitable for a plasticizer of a wrap film. To process a wrap film, the transfer resistance should not be too high or too low. The plasticizer of Example 1 offers a suitable transfer resistance for a wrap film.

And, the polyester resins using the diethylene glycol ester compositions of Examples 2 and 3 as plasticizer have comparable, although not significantly superior, physical properties, compared with those of Comparative Examples. These physical properties are adequate for a wrap film. More importantly, they do not comprise phthalate, adipate, or trimelitate compounds, which are known to generate environmental hormones.

As described above, the diethylene glycol ester based plasticizer composition of the present invention improves tensile strength, elongation, transfer resistance, hardness, transparency, adhesivity, and/or compatibility of a polyvinyl chloride resin without generating environmental hormones, when employed as the plasticizer of the resin.

While the present invention has been described in detail with reference to the preferred embodiments, those skilled in the art will appreciate that various modifications and substitutions can be made thereto without departing from the spirit and scope of the present invention as set forth in the appended claims.

**Claims**

**1.** A diethylene glycol ester based plasticizer composition comprising

(a) 10 to 80 wt% of benzoic acid 2-{2-(2-ethylhexanoyloxy)-ethoxy}-ethylester,
(b) 5 to 80 wt% of 2-ethylhexanoic acid 2-{2-(2-ethylhexanoyloxy)-ethoxy}-ethyl ester, and
(c) 5 to 60 wt% of benzoic acid 2-{2-(benzoyloxy)-ethoxy}-ethyl ester.

**2.** A method of preparing a diethylene glycol ester based plasticizer composition of claim 1 comprising the step of esterifying 10 to 40 wt% of diethylene glycol, 20 to 70 wt% of 2-ethylhexanoic acid, 10 to 60 wt% of benzoic acid, 1 to 10 wt% of xylene, and 0.01 to 1 wt% of tetraisopropyl titanate at 220°C for 4 to 10 hours.

**3.** The preparing method of claim 2, wherein the composition comprises:

(a) 10 to 80 wt% of benzoic acid 2-{2-(2-ethylhexanoyloxy)-ethoxy}-ethylester,
(b) 5 to 80 wt% of 2-ethylhexanoic acid 2-{2-(2-ethylhexanoyloxy)-ethoxy}-ethyl ester, and
(c) 5 to 60 wt% of benzoic acid 2-{2-(benzoyloxy)-ethoxy}-ethyl ester.

**4.** A polyvinyl chloride resin containing a diethylene glycol ester based plasticizer composition of claim 1.

**Patentansprüche**

**1.** Weichmacherzusammensetzung auf Basis von Diethylenglykolester, welche umfasst

(a) 10 bis 80 Gew.-% Benzoesäure-2-{2-(2-ethylhexanoyloxy)-ethoxy}-ethylester,
(b) 5 bis 80 Gew.-% 2-Ethylhexansäure-2-{2-(2-ethylhexanoyloxy)-ethoxy}-ethylester, und
(c) 5 bis 60 Gew.-% Benzoesäure-2-{2-(2-benzoyloxy)-ethoxy}-ethylester.

**2.** Verfahren zum Herstellen einer Weichmacherzusammensetzung auf Basis von Diethylenglykolester nach Anspruch 1, umfassend den Schritt eines Veresterns von 10 bis 40 Gew.-% Diethylenglykol, 20 bis 70 Gew.-% 2-Ethylhexansäure, 10 bis 60 Gew.-% Benzoesäure, 1 bis 10 Gew.-% Xylol und 0,01 bis 1 Gew.-% Tetraisopropyltitanat bei 220°C fiir 4 bis 10 Stunden.

**3.** Herstellungsverfahren nach Anspruch 2, wobei die Zusammensetzung umfasst:

(a) 10 bis 80 Gew.-% Benzoesäure-2-{2-(2-ethylhexanoyloxy)-ethoxy}-ethylester,

(b) 5 bis 80 Gew.-% 2-Ethylhexansäure-2-{2-(2-ethylhexanoyloxy)-ethoxy}-ethylester, und

(c) 5 bis 60 Gew.-% Benzoesäure-2-{2-(2-benzoyloxy)-ethoxy}-ethylester.

4. Polyvinylchloridharz enthaltend eine Weichmacherzusammensetzung auf Basis von Diethylenglykolester nach Anspruch 1.

**Revendications**

1. Une composition plastifiante basée sur de l'ester de diéthylène glycol consistant en

(a) 10 à 80% en poids de l'ester éthylique de l'acide benzoïque 2-{2-(2-éthylhexanoyloxy)-éthoxy},

(b) 5 à 80% en poids de l'ester éthylique de l'acide 2-éthylhexanoïque 2-{2-(2-éthylhexanoyloxy)-éthoxy}, et

(c) 5 à 60% en poids de l'ester éthylique de l'acide benzoïque 2-{2-(2-benzoyloxy)-éthoxy}.

2. Une méthode de préparation d'une composition plastifiante basée sur de l'ester de diéthylène glycol de la revendication 1 comprenant l'étape de d'estérification de 10 à 40% en poids de diéthylène glycol, de 20 à 70% en poids d'acide 2-éthylhexanoïque, de 10 à 60% en poids d'acide benzoïque, de 1 à 10% en poids de xylène et de 0,01 à 1% en poids de titanate de tétraisopropyle à 220°C pendant 4 à 10 heures.

3. Méthode de préparation de la revendication 2, la composition de laquelle consiste en :

(a) 10 à 80% en poids de l'ester éthylique de l'acide benzoïque 2-{2-(2-éthylhexanoyloxy)-éthoxy},

(b) 5 à 80% en poids de l'ester éthylique de l'acide 2-éthylhexanoïque 2-{2-(2-éthylhexanoyloxy)-éthoxy} et

(c) 5 à 60% en poids de l'ester éthylique de l'acide benzoïque 2-{2-(2-benzoyloxy)-éthoxy}.

4. Une résine de chlorure de polyvinyle contenant une composition plastifiante basée sur l'ester de diéthylène glycol de la revendication 1.

[Fig. 1]

# FIG.1

[Fig. 2]

# FIG.2

Abundance
1.5e+07
1.4e+07
1.3e+07
1.2e+07
1.1e+07
1e+07
9000000
8000000
7000000
6000000
5000000
4000000
3000000
2000000
1000000

TIC:0139103.D

B

Time→  2.00  4.00  6.00  8.00  10.00  12.00  14.00  16.00  18.00  20.00  22.00  24.00

Abundance  Average of 13.702 to 13.903 min.:0139103.D(-)
2000000
1800000
1600000
1400000
1200000
1000000
800000
600000
400000
200000

B

MW 336.43

This mass spectrum pattern is mexed with A and D

[Fig. 3]

# FIG.3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 5746783 A